# EUROPÄISCHE PATENTSCHRIFT

(11) **EP 3 124 060 B1**
(45) Veröffentlichungstag und Bekanntmachung des Hinweises auf die Patenterteilung: **11.09.2019**
(21) Anmeldenummer: 15178666.2
(22) Anmeldetag: 28.07.2015
(51) Int. Cl.: A61M 1/00

(54) **SCHALLDÄMPFER FÜR EINE UNTERDRUCKTHERAPIEEINHEIT**
SILENCER FOR A VACUUM THERAPY UNIT
AMORTISSEUR DE BRUIT POUR UNE UNITE DE THERAPIE A PRESSION NEGATIVE

(43) Veröffentlichungstag der Anmeldung: 01.02.2017
(73) Patentinhaber: Paul Hartmann AG, 89522 Heidenheim (DE)
(72) Erfinder: Beyrle, Karina, 89129 Langenau (DE); Croizat, Pierre, Dr., 89542 Herbrechtingen (DE); Klusmann, Johanna, 73450 Neresheim (DE); Grimwade, Stephen John, Huntingdon, Cambridgeshire PE26 2QQ (GB); Woodward, Adrian Michael, Bury St Edmunds, Suffolk IP32 7PG (GB); Stein, James Edward, Fulborn, Cambridge CB21 5DX (GB)

(56) Entgegenhaltungen:
- WO-A1-2009/004369
- WO-A2-03/101508
- DE-A1-102009 048 880

## Beschreibung

### Neue Beschreibung

Die Erfindung betrifft eine Unterdrucktherapieeinheit zur Behandlung von Wunden am menschlichen oder tierischen Körper mittels Unterdruck. Die Unterdrucktherapieeinheit umfasst ein die Unterdrucktherapieeinheit nach außen abgrenzendes Gehäuse, eine Pumpe zur Erzeugung eines Unterdrucks und einen Schalldämpfer für die Pumpe. Der Schalldämpfer weist ein Gehäuse, welches einen Hohlraum ausbildet, auf. Der Schalldämpfer weist weiterhin ein schalldämpfendes Material auf. Weiterhin betrifft die Erfindung eine Vorrichtung zur Unterdrucktherapie von Wunden am menschlichen oder tierischen Körper umfassend eine erfindungsgemäße Unterdrucktherapieeinheit.

Die Behandlung von Wunden am menschlichen oder tierischen Körper mittels Unterdruck ist im Stand der Technik bekannt, beispielsweise aus der internationalen Patentanmeldung WO93109727. Insbesondere wird die Unterdruckwundtherapie zur Behandlung von stark exsudierenden oder chronischen, schlecht heilenden Wunden der Haut angewandt. Im Allgemeinen wird bei der Unterdrucktherapie das zu behandelnde Wundareal mit einem Verband luftdicht gegenüber die die Wunde umgebende Atmosphäre abgedeckt. Bei tiefen Wunden der Haut wird hierfür häufig ein offenzelliger Schaumstoff auf Polyurethanbasis als Wundfüller und ein einseitig selbstklebender Folienverband als luftdichte Wundabdeckung eingesetzt. Mit Hilfe einer Unterdrucktherapieeinheit beziehungsweise eines Unterdrucktherapiegerätes und eines Leitungsmittels wird die Wunde einem Unterdruck ausgesetzt, um Wundflüssigkeit abzusaugen und die Wundheilung zu fördern. Bei dem Leitungsmittel handelt es sich im einfachsten Fall um einen Drainageschlauch, welcher an seinem einen Ende in den Wundverband geführt wird und an seinem anderen Ende mit einem zur Unterdrucktherapieeinheit gehörenden Sammelbehälter für die abgesaugte Wundflüssigkeit verbunden wird.

Unterdrucktherapieeinheiten sind kommerziell erhältlich, wie beispielsweise die VivanoTec®-Unterdrucktherapieeinheit des Anmelders (Paul Hartmann AG, Heidenheim, Deutschland). Viele kommerziell erhältliche Unterdrucktherapieeinheiten setzen als Unterdruckquelle eine elektrisch betriebene Pumpe wie zum Beispiel eine Membranpumpe ein. Derartige elektrisch betriebene Unterdruckquellen sind leistungsfähig und gut steuerbar. Sie können jedoch während der Unterdrucktherapie für die Patienten und für das Pflegepersonal eine Lärmbelästigung darstellen, wenn sie mit einer hohen Leistung betrieben werden. Aus diesem Grund besitzen Unterdrucktherapieeinheiten mit einer elektrischen Unterdruckquelle zumeist einen Schalldämpfer. Die Schalldämpfer sollen die Pumpengeräusche reduzieren, so dass die Patienten und das Pflegepersonal während der Unterdrucktherapie keiner Lärmbelästigung ausgesetzt sind. Es besteht das Bedürfnis, die schalldämpfenden Komponenten hinsichtlich ihrer Einbindung in die Unterdrucktherapiesysteme und hinsichtlich ihrer Leistungsfähigkeit weiter zu verbessern.

Die DE102009048880A1 beschreibt ein medizinisches Absauggerät mit einer abluftseitig zur Atmosphäre hin offenen Unterdruckpumpe zum Erzeugen eines patientenseitigen Unterdrucks zum Absaugen von Körperflüssigkeiten. Das Absauggerät ist dadurch gekennzeichnet, dass die Ein- und Ausgänge der Unterdruckpumpe direkt und ohne Verbindungsschläuche mit den Ein- und Ausgängen einer pneumatischen Anschlussbox verbunden sind, wobei im Inneren der Anschlussbox durch Trennwände pneumatische Leitungskanäle und Funktionskammern ausgebildet sind.

Die WO2009/004369 offenbart eine Unterdrucktherapieeinheit zur Wundbehandlung mit einem Schalldämpfer, welcher ein schalldämpfendes Material und eine einzige Filterkammer umfasst. Ein rechteckiges, kastenartiges Gehäuseteil des Schalldämpfers ist an einer inneren Rahmenstruktur der Unterdrucktherapieeinheit befestigt und wird von einem plattenartigen Deckel verschlossen.

Die WO03/101508 beschreibt ein Gerät zur kombinierten Unterdruck-und Instillationstherapie von Wunden.

Der vorliegenden Erfindung liegt die Aufgabe zugrunde, die Unterdrucktherapie von Wunden weiter zu verbessern und die Nachteile im Stand der Technik zu überwinden. Insbesondere liegt der vorliegenden Erfindung die Aufgabe zugrunde, einen Schalldämpfer für eine Unterdrucktherapieeinheit mit einem verbesserten Aufbau und einem verbesserten schalldämpfenden Effekt bereitzustellen. Weiterhin liegt der Erfindung die Aufgabe zugrunde, einen Schalldämpfer einfach, effizient und stabil in einer Unterdrucktherapieeinheit unterbringen zu können.

Die Erfindung löst die genannten Aufgaben mit einer Unterdrucktherapieeinheit mit den Merkmalen von Anspruch 1 und einer Vorrichtung mit den Merkmalen von Anspruch 15. Der Schalldämpfer der erfindungsgemäßen Unterdrucktherapieeinheit zeichnet sich durch eine treppenartig gestufte Außenfläche und mehrere im Wesentlichen quaderförmige, aneinander angrenzende Filterkammern unterschiedlicher Größe aus. Dieser spezielle Aufbau kann dem Schalldämpfer verbesserte geräuschdämpfende Eigenschaften verleihen.

Die Erfindung betrifft demnach gemäß einem ersten Aspekt eine Unterdrucktherapieeinheit zur Behandlung von Wunden am menschlichen oder tierischen Körper mittels Unterdruck nach Anspruch 1.

Weiterhin betrifft die Erfindung gemäß einem zweiten Aspekt eine Vorrichtung zur Unterdrucktherapie von Wunden am menschlichen oder tierischen Körper nach Anspruch 15.

Die erfindungsgemäße Unterdrucktherapieeinheit wird von einem Gehäuse nach außen abgegrenzt. Üblicherweise besteht das Gehäuse aus mehreren Teilen und wird im letzten Herstellungsschritt der Unterdrucktherapieeinheit zusammengefügt. Die Form und die Größe des Gehäuses können sehr unterschiedlich sein. Wie in den beiden internationalen Patentanmeldungen WO2011/018132 und WO2011/018133 des Anmelders dargestellt, kann das Gehäuse einer Unterdrucktherapieeinheit beispielsweise oval und flach ausgebildet sein und an der Rückseite eine Anschlussmöglichkeit für einen Behälter zum Sammeln der abgesaugten Wundflüssigkeit aufweisen. Neben der Pumpe und dem Schalldämpfer befinden sich an dem Gehäuse oder innerhalb des Gehäuses normalerweise noch eine Vielzahl weiterer, für den Betrieb der Unterdrucktherapieeinheit erforderliche Komponenten (zum Beispiel ein Akkumulator, eine Anzeige, Bedienelemente, Leiterplatten, Sensoren, Ventile).

Bei der von der erfindungsgemäßen Unterdrucktherapieeinheit umfassten Pumpe handelt es sich insbesondere um eine elektrisch betriebene Pumpe, zum Beispiel um eine Membranpumpe. Der Eingang der Pumpe, über welchen Fluide angesaugt werden können, ist dafür vorgesehen mit der zu behandelnden Wunde in eine fluide Kommunikation gebracht zu werden. Hierfür wird der Eingang der Pumpe üblicherweise mit einem der Pumpe vorgeschalteten Sauganschluss, welcher sich an einer Außenfläche der Unterdrucktherapieeinheit befindet, in fluide Kommunikation gebracht. Der Ausgang der Pumpe steht in fluider Kommunikation mit dem Eingang des der Pumpe nachgeschalteten Schalldämpfers. Der Ausgang des Schalldämpfers steht wiederum in fluider Kommunikation mit der die Unterdrucktherapieeinheit umgebenden Atmosphäre. Die den Schalldämpfer verlassende Abluft kann ohne weitere Vorkehrungen in das Innere der Unterdrucktherapieeinheit entlassen werden, von wo aus sie über Fugen beziehungsweise luftdurchlässige Verbindungsstellen des Gehäuses der Unterdrucktherapieeinheit nach außen entweichen kann. Die den Schalldämpfer verlassende Abluft kann auch in die Nähe von oder unmittelbar zu einem Auslass der Unterdrucktherapieeinheit geleitet werden. Dieser Auslass kann beispielsweise in der Form von schlitzartigen Öffnungen in dem Gehäuse der Unterdrucktherapieeinheit ausgebildet sein. Um die zuvor beschriebene fluide Kommunikation zwischen dem Sauganschluss, der Pumpe, dem Schalldämpfer und dem Auslass herzustellen, werden normalerweise Kunststoffschläuche als Leitungsmittel verwendet. Entsprechend kann ein erster Schlauch den Sauganschluss mit dem Eingang der Pumpe, ein zweiter Schlauch den Ausgang der Pumpe mit dem Eingang des Schalldämpfers und ein dritter Schlauch den Ausgang des Schalldämpfers mit dem Auslass verbinden.

Erfindungsgemäß umfasst das Gehäuse des Schalldämpfers einen Hohlkörper in der Form eines geraden Prismas mit einer Grundfläche, einer Deckfläche und einer Mantelfläche. Die Grundfläche des Prismas wird auf Basis eines rechtwinkligen Dreiecks mit einer ersten Kathete, einer zweiten Kathete und einer imaginären Hypotenuse gebildet. Die erste und die zweite Kathete bilden Seiten der Grundfläche. Die Hypotenuse hingegen ist keine Seite der Grundfläche und wird deshalb als imaginäre Hypotenuse bezeichnet. Die Grundfläche weist entlang der imaginären Hypotenuse eine treppenartige Stufung auf. Die Stufung ist daher mit einem Anstieg, welcher im Wesentlichen dem Verlauf der imaginären Hypotenuse entspricht, ausgebildet. Die die treppenartige Stufung bildenden Abschnitte (Seiten) der Grundfläche verlaufen entweder parallel oder rechtwinklig zu der ersten oder der zweiten Kathete des Dreiecks, auf welchem die Grundfläche basiert. Infolge der treppenartigen Stufung handelt es sich bei der Grundfläche des Prismas um ein Vieleck mit mindestens sechs Eckpunkten. Alternativ kann die Grundfläche des Prismas auch als ein orthogonales Vieleck mit mindestens sechs Ecken beschrieben werden. Das Vieleck hat eine erste Seite, eine zweite Seite und eine dritte Seite, wobei die dritte Seite eine treppenartige Stufung aufweist. In das Vieleck kann ein rechtwinkliges Dreieck gemäß der folgenden Maßgaben eingepasst werden. Gemäß der ersten Maßgabe bilden die erste und die zweite Seite der Grundfläche die Katheten des in die Grundfläche eingepassten Dreiecks. Gemäß der zweiten Maßgabe verläuft die treppenartige Stufung der dritten Seite der Grundfläche entlang der Hypotenuse des in die Grundfläche eingepassten Dreiecks. Alternativ kann die Grundfläche des Prismas weiterhin auch als ein aus mehreren Rechtecken zusammengesetztes Vieleck beschrieben werden. Die Rechtecke sind unterschiedlich dimensioniert und werden zur Ausbildung der Grundfläche in aufsteigender Größe auf einer Geraden ohne sich dabei zu überlagern aneinandergereiht. Die aneinandergereihten Rechtecke bilden eine treppenartige Stufung aus. Treppenartig bedeutet im vorliegenden Zusammenhang, dass die Stufung einer Treppe ähnelnd ausgebildet ist. Bei einem orthogonalen Vieleck treffen alle Kanten in einem rechten Winkel aufeinander, so dass der Innenwinkel an jeder Kante entweder 90° oder 270° beträgt.

Die Mantelfläche des Prismas weist dann eine nicht-gestufte erste Seitenfläche, eine nicht-gestufte zweite Seitenfläche und eine gestufte dritte Seitenfläche auf. Eine oder mehrere Trennwände, welche parallel zu einer der beiden nicht-gestuften Seitenflächen des Prismas und entlang einer Kante der dritten Seitenfläche des Prismas verlaufen, sind in dem Hohlraum des Prismas angeordnet. Die nicht-gestufte Seitenfläche des Prismas, welche parallel zu jeder Trennwand verläuft, wird im vorliegenden Dokument als erste Seitenfläche bezeichnet. Die nicht-gestufte Seitenfläche des Prismas, welche vertikal zu jeder Trennwand verläuft, wird im vorliegenden Dokument als zweite Seitenfläche bezeichnet.

Der Schalldämpfer weist dann im Wesentlichen quaderförmige, aneinander angrenzende Filterkammern unterschiedlicher Größe auf. Insbesondere weist der Schalldämpfer dann ausschließlich im Wesentlichen quaderförmige, aneinander angrenzende Filterkammern unterschiedlicher Größe auf.

Vorzugsweise ist die treppenartige Stufung der Grundfläche regelmäßig ausgebildet, so dass die einzelnen Stufen im Wesentlichen gleichmäßig dimensioniert sind. Insbesondere bildet die treppenartige Stufung der Grundfläche einen im Wesentlichen gleichmäßigen Anstieg, wobei die einzelnen Stufen im Wesentlichen gleichmäßig dimensioniert sind. Bevorzugt befindet sich zudem jeweils ein Eckpunkt der die treppenartige Stufung bildenden Abschnitte der Grundfläche auf oder in unmittelbarer Nähe zu der imaginären Hypotenuse.

Bevorzugt weist der Hohlraum des Schalldämpfers eine Trennwand bis zehn Trennwände, mehr bevorzugt eine Trennwand bis fünf Trennwände und besonders bevorzugt zwei Trennwände auf. Entsprechend weist der Schalldämpfer bevorzugt zwei bis elf Filterkammern, mehr bevorzugt zwei bis sechs Filterkammern und besonders bevorzugt drei Filterkammern auf. Ein Schalldämpfer mit drei Filterkammern lässt sich einfach herstellen und weist gute schalldämpfende Eigenschaften auf.

Weist die dritte Seitenfläche des Prismas eine Stufenanzahl (n) auf, so sind im Hohlraum des Prismas bevorzugt (n - 1) Trennwände angeordnet. Ganz besonders bevorzugt weist die dritte Seitenfläche des Prismas drei Stufen und der Hohlraum des Prismas zwei Trennwände auf, so dass drei Filterkammern ausgebildet werden können. Ebenso sind die Seitenkanten des Prismas (was der Höhe des Prismas entspricht) bevorzugt länger als die beiden nicht-gestuften Grundkanten des Prismas. Dabei ist die Länge jeder nicht-gestuften Grundkante einzeln zu betrachten.

In einer bevorzugten Ausführungsform der Erfindung ist an das Gehäuse der Unterdrucktherapieeinheit ein Behälter zum Sammeln von einer abgesaugten Wundflüssigkeit lösbar anbringbar. Der Behälter kann in fluide Kommunikation mit dem Eingang der Pumpe gebracht werden. Der Behälter soll die abgesaugte Wundflüssigkeit auffangen, so dass diese nicht in die Pumpe eindringen kann. Vorzugsweise umfasst der Behälter einen Eingang und einen Ausgang, wobei der Eingang in eine fluide Kommunikation mit einer zu behandelnden Wunde gebracht werden kann und der Ausgang in eine fluide Kommunikation mit dem Eingang der Pumpe gebracht werden kann. Der Behälter kann weiterhin einen Filter umfassen, welcher sich bei Kontakt mit einer Flüssigkeit verschließt und beispielsweise im Bereich des Behälterausgangs positioniert ist. Der Behälterausgang kann weiterhin an den zuvor beschriebenen Sauganschluss der Unterdrucktherapieeinheit gekoppelt sein, wenn der Behälter an das Gehäuse der Unterdrucktherapieeinheit angeschlossen ist. Derartige Sammelbehälter sind im Stand der Technik bekannt, unter anderem aus der WO2014/177544 und der WO2014/177545 des Anmelders. Alternativ zu dem Sammelbehälter kann ein absorbierender Wundverband vorgesehenen sein, welcher die Wundflüssigkeit speichert und verhindert, dass diese in die Pumpe eindringt.

Das Gehäuse des Schalldämpfers umfasst erfindungsgemäß einen Hohlkörper in der Form eines geraden Prismas. Dieser Hohlkörper kann ein erstes Gehäuseteil und ein zweites Gehäuseteil, welche zur Ausbildung der Filterkammern miteinander verbindbar sind beziehungsweise verbunden werden müssen, umfassen. Insbesondere kann der Hohlkörper aus einem ersten und einem zweiten Gehäuseteil bestehen. Die Gehäuseteile, welche nach dem Zusammenfügen den prismaförmigen Hohlkörper ausbilden, können sehr unterschiedlich ausgestaltet sein. Bevorzugt wird jedoch die zweite Seitenfläche der Mantelfläche des Prismas von dem ersten Gehäuseteil gebildet. Die Grundfläche des Prismas, die Deckfläche des Prismas, die erste Seitenfläche der Mantelfläche des Prismas, die dritte Seitenfläche der Mantelfläche des Prismas und jede Trennwand können dann von dem zweiten Gehäuseteil gebildet werden. Das zweite Gehäuseteil gemäß der zuletzt beschriebenen Ausgestaltung weist also eine offene zweite Seitenfläche auf, welche von dem entsprechend geformten ersten Gehäuseteil verschlossen werden kann. Die offene Außenfläche ist erforderlich, um die Filterkammern mit dem schalldämpfenden Material ausstatten zu können. Insofern nicht anders angegeben, wird im vorliegenden Dokument bei einem Verweis auf das erste Gehäuseteil oder das zweite Gehäuseteil stets auf den Schalldämpfer beziehungsweise dessen prismaförmigen Hohlkörper Bezug genommen.

In einer weiteren Ausführungsform der Erfindung umfasst das erste Gehäuseteil eine oder mehrere Erhebungen, insbesondere linienartige Erhebungen, zur Umrandung des zweiten Gehäuseteils und/oder zur Einpassung in das zweite Gehäuseteil. Die Erhebungen bilden in ihrer Form beziehungsweise in ihrem Formverlauf die äußere und/oder innere Peripherie der offenen Außenfläche des zweiten Gehäuseteils nach. Die Erhebungen können den Verschluss des zweiten Gehäuseteils mit dem ersten Gehäuseteil erleichtern.

Das erste Gehäuseteil und/oder das zweite Gehäuseteil können zudem ein Mittel umfassen, welches zur Befestigung der Gehäuseteile innerhalb der Unterdrucktherapieeinheit vorgesehen ist und/oder zur Befestigung anderer innerer Komponenten der Unterdrucktherapieeinheit vorgesehen ist und/oder eine Führung für ein Befestigungsmittel bildet. Zum Beispiel kann das zweite Gehäuseteil eine Führung für eine Schraube, welche Gehäuseteile der Unterdrucktherapieeinheit miteinander verbindet, umfassen.

Um die fluide Kommunikation zwischen dem Eingang und dem Ausgang des Schalldämpfers herzustellen, kann der Schalldämpfer zusätzliche Eingänge und Ausgänge aufweisen. Jede Filterkammer weist dann einen Eingang und einen Ausgang auf. Die zusätzlichen Eingänge und Ausgänge können mit Hilfe von Schläuchen fluidleitend miteinander verbunden werden. Insbesondere bei einer hohen Anzahl von Filterkammer führt diese Variante gegebenenfalls zu einer komplizierten Verschlauchung am Schalldämpfer. Um eine komplizierte Verschlauchung zu vermeiden, kann jede Trennwand gemäß einer alternativen Ausgestaltung eine Öffnung aufweisen, damit die Filterkammern innerhalb des Schalldämpfers in eine fluide Kommunikation miteinander gebracht werden können. Bei dieser alternativen Ausgestaltung sind weiterhin nur ein Eingang und ein Ausgang für den Schalldämpfer erforderlich. Es werden somit keine zusätzlichen Leitungsmittelabschnitte benötigt, um den mehrere Filterkammern umfassenden Schalldämpfer in den Fluidpfad einzubinden.

Vorzugsweise wird jeder Eingang des Schalldämpfers und jeder Ausgang des Schalldämpfers von dem zuvor beschriebenen, eine offene Außenfläche aufweisenden zweiten Gehäuseteil des Schalldämpfers gebildet. Besonders bevorzugt sind jeder Eingang des Schalldämpfers, jeder Ausgang des Schalldämpfers und falls vorhanden jede Öffnung einer Trennwand derart zueinander positioniert, dass ein verschlungener Fluidpfad durch die Filterkammern des Schalldämpfers ausgebildet werden kann. Unter einem verschlungenen Fluidpfad ist insbesondere ein Fluidpfad mit mindestens einem Richtungswechsel von 180° für die den Schalldämpfer durchströmenden Fluide zu verstehen. Der verschlungene Fluidpfad erhöht die Länge des Fluidpfades innerhalb des Schalldämpfers. Dadurch werden die schalldämpfenden Eigenschaften des Schalldämpfers verbessert.

Der Schalldämpfer kann für jede Filterkammer einen Eingang und einen Ausgang aufweisen. Diese sind dann vorzugsweise an zwei gegenüberliegenden Außenflächen des Prismas angeordnet, wobei es sich bei den gegenüberliegenden Außenflächen des Prismas um die Grundfläche des Prismas und die Deckfläche des Prismas handelt.

Alternativ kann der Schalldämpfer nur einen Eingang, nur einen Ausgang und eine Öffnung in jeder Trennwand aufweisen. Der Eingang und der Ausgang des Schalldämpfers sind dann jeweils an der Grundfläche oder der Deckfläche des Prismas angeordnet und münden jeweils in eine der beiden äußeren Filterkammern. Das heißt, der Eingang und der Ausgang können an nur einer der beiden vorgenannten Flächen angeordnet sein (im Falle einer geraden Anzahl von Filterkammer) oder versetzt gegenüberliegend an der Grundfläche und der Deckfläche angeordnet sein (im Falle einer ungeraden Anzahl von Filterkammern), aber sie münden nicht in dieselbe Filterkammer. Ein zweistufiger Schalldämpfer weist nur äußere Filterkammern auf. Weiterhin grenzt die Öffnung jeder Trennwand an die Grundfläche oder die Deckfläche des Prismas an. Der Eingang des Schalldämpfers, die Öffnung in der Trennwand, falls vorhanden die Öffnung jeder weiteren Trennwand und der Ausgang des Schalldämpfers sind derart gegenüberliegend an der Grundfläche und der Deckfläche des Prismas angeordnet beziehungsweise grenzen derart gegenüberliegend an die Grundfläche und die Deckfläche des Prismas an, dass ein verschlungener Fluidpfad durch die Filterkammern des Schalldämpfers ausgebildet werden kann.

Gemäß einer weiteren, besonders bevorzugten Ausgestaltung des Schalldämpfers wird die Öffnung in jeder Trennwand dadurch ausgebildet, dass die Trennwand nicht durchgehend zwischen der Grundfläche und der Deckfläche des Prismas verläuft. Es verbleibt daher ein Spalt zwischen der Trennwand und der Grundfläche oder der Deckfläche des Prismas. Dieser Spalt stellt die Öffnung dar. Der Spalt sollte idealerweise nur so groß ausgebildet sein, dass einerseits eine ungehinderte fluide Kommunikation mit der angrenzenden Filterkammer hergestellt werden kann, andererseits aber die Länge des Fluidpfades im Schalldämpfer nicht wesentlich verkleinert wird. Die Länge der nicht-durchgehenden Trennwand beträgt daher bevorzugt mindestens etwa 90 %, mehr bevorzugt mindestens etwa 95 % und besonders bevorzugt etwa 97 % der Länge der entsprechenden durchgehend verlaufenden Trennwand ohne Spalt.

Weiterhin werden die verschiedenen Filterkammern des Schalldämpfers vorzugsweise in Richtung abnehmende Filterkammergröße von der Abluft der Pumpe durchströmt. Die Abluft der Pumpe wird also vorzugsweise zunächst in die größte Filterkammer des Schalldämpfers eingeleitet und schließlich über die kleinste Filterkammer nach Außen abgegeben. Im Falle des nur einen Eingang und nur einen Ausgang aufweisenden Schalldämpfers münden folglich der Eingang bevorzugt in die größte Filterkammer und der Ausgang bevorzugt in die kleinste Filterkammer.

Allgemein sind die Eingänge und die Ausgänge des Schalldämpfers als Öffnungen im Schalldämpfergehäuse zu verstehen. Vorzugsweise ist jedoch jeder Eingang und jeder Ausgang des Schalldämpfers auch mit einem Schlauchanschluss ausgestattet. Die Schlauchanschlüsse können im Wesentlichen zylinderförmig ausgestaltet sein. Schläuche können dann an den Eingängen und den Ausgängen des Schalldämpfers befestigt werden, indem ihre Endabschnitte auf die Schlauchanschlüsse geschoben werden. Derartige Schlauchanschlüsse sind im Stand der Technik bekannt und üblicherweise auch an vielen weiteren Komponenten der Unterdrucktherapieeinheit wie zum Beispiel der Pumpe vorhanden.

Die Unterdrucktherapieeinheit kann weiterhin einen Rahmen zur Anordnung und/oder Befestigung von inneren Komponenten der Unterdrucktherapieeinheit umfassen. Der Rahmen befindet sich innerhalb des Gehäuses der Unterdrucktherapieeinheit. Ebenso wie das Gehäuse der erfindungsgemäßen Unterdrucktherapieeinheit kann der Rahmen unterschiedlich ausgestaltet sein. Allgemein ist unter dem Begriff Rahmen im vorliegenden Dokument eine Struktur innerhalb der Unterdrucktherapieeinheit zu verstehen, welche zur Anordnung und/oder Befestigung von inneren Komponenten der Unterdrucktherapieeinheit vorgesehen ist. Der Rahmen kann die Oberfläche im Inneren der Unterdrucktherapieeinheit vergrößern und den Raum im Inneren der Unterdrucktherapieeinheit in verschiedene Bereiche aufgliedern. Der Rahmen stellt also eine zusätzliche Oberfläche zur Verfügung, an der innere Komponenten der Unterdrucktherapieeinheit angeordnet und befestigt werden können. Die Ausgestaltung des Rahmens richtet sich beispielsweise nach der Form und der Größe der Unterdrucktherapieeinheit sowie der Anzahl, der Form und der Größe der darin zu verstauenden Gerätekomponenten. Es können eine oder auch mehrere derartige Strukturen im Inneren der Unterdrucktherapieeinheit vorhanden sein. Vorzugsweise handelt es sich bei dem Rahmen um ein einteiliges Formstück, welches zum Beispiel an einer Innenseite des Gehäuses der Unterdrucktherapieeinheit befestigt wird. Der Rahmen kann auch dazu genutzt werden, den Schalldämpfer innerhalb des Gehäuses der Unterdrucktherapieeinheit anzuordnen und zu befestigen. Beispielsweise kann das erste und/oder das zweite Gehäuseteil mit einer Oberfläche des Rahmens verklebt werden.

In einer besonders bevorzugten Ausführungsform der Erfindung wird das erste Gehäuseteil des Schalldämpfers durch einen Abschnitt des Rahmens gebildet, so dass zur Ausbildung der Filterkammern der das erste Gehäuseteil bildende Rahmenabschnitt mit dem zweiten Gehäuseteil verbunden werden muss. Eines der beiden Gehäuseteile des Schalldämpfers, nämlich das erste Gehäuseteil, ist nach dieser Ausführungsform also in den Rahmen integriert und liegt nicht als von dem Rahmen losgelöste, separate Komponente vor. Um die Filterkammern des Schalldämpfers auszubilden, ist daher die Anbindung des zweiten Gehäuseteils an den Rahmen erforderlich. Durch die Integrierung des ersten Gehäuseteils in den Rahmen kann der Schalldämpfer einfach, effizient und stabil in der Unterdrucktherapieeinheit untergebracht werden.

Der Rahmen kann eine erste, eine zweite und eine dritte Platte umfassen. Die erste und die zweite Platte verlaufen im Wesentlichen parallel zueinander und befinden sich im Wesentlichen in Deckung zueinander. Die dritte Platte verläuft im Wesentlichen vertikal zu der ersten und der zweiten Platte und verbindet die erste Platte im Wesentlichen mittig mit der zweiten Platte, so dass ein doppel-T-förmiger Rahmenabschnitt ausgebildet wird. Der doppel-T-förmige Rahmenabschnitt ist derart in dem Gehäuse der Unterdrucktherapieeinheit angeordnet, dass die erste und die zweite Platte im Wesentlichen parallel zu einer horizontalen Unterlage, auf welche die Unterdrucktherapieeinheit ihre bestimmungsgemäße Standposition einnimmt, verlaufen. Die erste Platte ist der Unterseite und die zweite Platte ist der Oberseite der Unterdrucktherapieeinheit zugewandt. Ein Abschnitt der zweiten Platte kann das erste Gehäuseteil des Schalldämpfers bilden und das zweite Gehäuseteil des Schalldämpfers ist dann mit der zur Oberseite der Unterdrucktherapieeinheit gewandten Seite der zweiten Platte verbunden. Zudem sind die beiden, von der ersten, der zweiten und der dritten Platte begrenzten Bereiche vorzugsweise zur Anordnung beziehungsweise Verstauung eines Akkumulators und der Pumpe vorgesehen. Die mit der Pumpe in Kontakt stehende Oberfläche des Rahmens wird dabei bevorzugt möglichst klein gehalten, zum Beispiel indem Segmente des Rahmens ausgeschnitten oder schmale Rippen auf der Rahmenoberfläche angeordnet werden. Weiterhin können der Akkumulator und die Pumpe von einer Schaumstofflage im Rahmen eingefasst sein. Die verkleinerte Rahmenoberfläche sowie der Schaumstoff können die Pumpengeräusche zusätzlich reduzieren.

Der Schalldämpfer der erfindungsgemäßen Unterdrucktherapieeinheit umfasst ein schalldämpfendes Material. Das schalldämpfende Material füllt die Filterkammern des Schalldämpfers bevorzugt teilweise oder vollständig aus. Insbesondere ist jede Filterkammer des Schalldämpfers vollständig mit dem schalldämpfenden Material ausgefüllt. Bei dem schalldämpfenden Material kann es sich beispielsweise um einen Schaumstoff, insbesondere um einen offenporigen Schaumstoff, handeln. Besonders bevorzugt ist jede Filterkammer des Schalldämpfers vollständig mit einem Schaumstoff, insbesondere mit einem offenporigen Schaumstoff, als schalldämpfendem Material ausgefüllt. Ein geeigneter offenporiger Schaumstoff ist in der WO2012/022485 des Anmelders offenbart. Es ist auch vorstellbar als schalldämpfendes Material Mineralwolle oder ein anderes Fasermaterial auf Basis von Baumwollfasern, Glasfasern, Kunststofffasern oder Mischungen davon einzusetzen.

Das Gehäuse des Schalldämpfers, das erste Gehäuseteil, das zweite Gehäuseteil und/oder der Rahmen können ein Kunststoffmaterial umfassen oder aus einem Kunststoffmaterial bestehen. Ein geeignetes Kunststoffmaterial für das Schalldämpfergehäuse und den Rahmen ist beispielsweise Acrylnitril-Butadien-Styrol (ABS). Ein besonders geeignetes Acrylnitril-Butadien-Styrol ist Terluran® GP-22 (Styrolution, Frankfurt am Main, Deutschland). Weiterhin können das erste und das zweite Gehäuseteil durch einen Klebstoff, durch Ultraschallschweißen oder durch Quellschweißen miteinander verbunden werden. Besonders bevorzugt werden das erste und das zweite Gehäuseteil durch einen Klebstoff, insbesondere einen Klebstoff umfassend Ethyl-2-cyanacrylat (zum Beispiel Loctite® 4061 Med Liquid Adhesive von der Firma Henkel), miteinander verbunden.

Die vorliegende Erfindung betrifft gemäß dem zweiten Aspekt eine Vorrichtung zur Unterdrucktherapie von Wunden am menschlichen oder tierischen Körper. Die Vorrichtung umfasst eine Unterdrucktherapieeinheit nach dem ersten Aspekt der Erfindung.

Weiterhin umfasst die Vorrichtung einen Wundverband. Der Wundverband schließt die Wunde im Wesentlichen luftdicht gegenüber die den Wundverband umgebende Atmosphäre ab, so dass ein Unterdruck innerhalb des Wundverbandes hergestellt und aufrechterhalten werden kann. Als Unterdruck ist dabei ein gegenüber dem den Wundverband umgebenden Luftdruck (Umgebungsluftdruck) erniedrigter Luftdruck innerhalb des Wundverbandes zu verstehen. Üblicherweise wird bei der Unterdrucktherapie von Wunden der Druck innerhalb des Wundverbandes um höchstens 250 mmHg gegenüber dem Umgebungsluftdruck erniedrigt. Geeignete Materialien für einen derartigen Unterdruckwundverband sind im Stand der Technik bekannt und kommerziell erhältlich, wie zum Beispiel die Produkte "VivanoMed Foam®" und "Hydrofilm®" (Paul Hartmann AG, Heidenheim, Deutschland).

Die Vorrichtung umfasst auch ein Verbindungsmittel, welches eine fluide Kommunikation zwischen der Unterdrucktherapieeinheit und dem Wundverband herstellen kann. Bei dem Verbindungsmittel kann es sich um einen Drainageschlauch handeln, welcher an seinem wundseitigen Ende mit Hilfe eines Anschlussstücks (Port) an dem Wundverband befestigt wird und an seinem geräteseitigen Ende an dem Eingang eines an der Unterdrucktherapieeinheit angekoppelten Sammelbehälters angeschlossen wird. Ein zu diesem Zweck geeignetes Anschlussstück ist beispielsweise in der WO2011076340 des Anmelders offenbart und unter der Bezeichnung "VivanoTec Port®" (Paul Hartmann AG, Heidenheim, Deutschland) im Handel erhältlich.

Die zuvor beschriebenen Merkmale der Unterdrucktherapieeinheit in den bevorzugten Ausführungsformen können auf die Vorrichtung nach dem zweiten Aspekt der Erfindung entsprechend übertragen werden.

### Figuren

Nachstehend werden die Aspekte der Erfindung anhand von Zeichnungen sowie Messdaten näher erläutert. Die Erfindung soll nicht auf die in den Zeichnungen oder in der Beschreibung der Zeichnungen dargestellten Ausgestaltungen reduziert verstanden werden. Vielmehr umfasst die Erfindung auch Kombinationen der Einzelmerkmale der alternativen Formen.
Figur 1 zeigt eine Vorrichtung zur Unterdrucktherapie gemäß des zweiten Aspektes schematisch in einer bevorzugten Ausführungsform.
Figur 2 zeigt ein erstes Gehäuseteil des Schalldämpfers in einer bevorzugten Ausführungsform.
Figuren 3a bis 3e zeigen ein zweites Gehäuseteil des Schalldämpfers in einer bevorzugten Ausführungsform.
Figuren 4a bis 4c zeigen ein zweites Gehäuseteil des Schalldämpfers in einer weiteren bevorzugten Ausführungsform.
Figur 5 zeigt das erste Gehäuseteil nach Figur 2 und das zweite Gehäuseteil nach Figur 3 vor dem Verbinden der Gehäuseteile.
Figuren 6 und 7 zeigen ein in einem Rahmen integriertes erstes Gehäuseteil und das zweite Gehäuseteil nach Figur 3 vor beziehungsweise nach dem Verbinden der Gehäuseteile.
Figur 8 zeigt den Rahmen mit dem Schalldämpfer nach Figur 7 und weitere Komponenten der Unterdrucktherapieeinheit in einer bevorzugten Ausführungsform.
Figuren 9 und 10 zeigen die Unterdrucktherapieeinheit in einer bevorzugten Ausführungsform mit den Komponenten aus Figur 8 und mit geöffnetem beziehungsweise verschlossenem Gehäuse.
Figur 11 zeigt die Unterdrucktherapieeinheit nach Figur 10 mit einem angekoppelten Sammelbehälter für die abgesaugte Wundflüssigkeit in einer bevorzugten Ausführungsform.
Figur 12 zeigt Messdaten von Schalldämpfern für Unterdrucktherapieeinheiten.

### Figurenlegende

- 1: Unterdrucktherapieeinheit
- 2: Gehäuse der Unterdrucktherapieeinheit
- 3: Pumpe der Unterdrucktherapieeinheit
- 4: Eingang der Pumpe
- 5: Ausgang der Pumpe
- 6: Schalldämpfer der Unterdrucktherapieeinheit
- 7: Gehäuse des Schalldämpfers
- 8: Eingang des Schalldämpfers
- 9: Ausgang des Schalldämpfers
- 10: Filterkammer des Schalldämpfers
- 11: Rahmen der Unterdrucktherapieeinheit
- 12: Auslass der Unterdrucktherapieeinheit
- 13: Sammelbehälter der Unterdrucktherapieeinheit
- 14: Eingang des Sammelbehälters
- 15: Ausgang des Sammelbehälters
- 16: Körperteil eines Patienten mit einer Wunde
- 17: Wundverband der Vorrichtung
- 18: Wunde
- 19: Verbindungsmittel der Vorrichtung
- 20: Anschlussstück (Port) des Verbindungsmittels
- 21: erste Gehäuseteil des Schalldämpfers
- 22: Platte des ersten Gehäuseteils
- 23: Erhebungen des ersten Gehäuseteils
- 24: zweite Gehäuseteil des Schalldämpfers
- 25: Grundfläche des zweiten Gehäuseteils
- 26: Deckfläche des zweiten Gehäuseteils
- 27: erste Kathete des der Grundfläche zugrunde liegenden Dreiecks
- 28: zweite Kathete des der Grundfläche zugrunde liegenden Dreiecks
- 29: imaginäre Hypotenuse des der Grundfläche zugrunde liegenden Dreiecks
- 30: nicht-gestufte erste Seitenfläche des zweiten Gehäuseteils
- 31: nicht-gestufte zweite Seitenfläche des zweiten Gehäuseteils (offen)
- 32: gestufte dritte Seitenfläche des zweiten Gehäuseteils
- 33: Trennwände des zweiten Gehäuseteils
- 34: Kanten des zweiten Gehäuseteils, an die die Trennwände anschließen
- 35: Öffnungen in den Trennwänden (Spalt)
- 36: ergänzende Gehäusekomponente des zweiten Gehäuseteils (Befestigungsmittel)
- 37: in einem Rahmen integriertes erstes Gehäuseteil
- 38: erste Platte des Rahmens
- 39: zweite Platte des Rahmens
- 40: dritte Platte des Rahmens
- 41: Bereich des Rahmens zur Anordnung des Akkumulators
- 42: Bereich des Rahmens zur Anordnung der Pumpe
- 43: Ausgeschnittenes Segment des Rahmens
- 44: Rippen des Rahmens
- 45: schalldämpfende Material der Filterkammern
- 46: Schaumstofflagen des Rahmens
- 47: Abdeckung des Rahmens
- 48: Akkumulator der Unterdrucktherapieeinheit
- 49: Leiterplatte
- 50: Schaumstofflage zur Befestigung am rückseitigen Gehäuseteil der Unterdrucktherapieeinheit
- 51: rückseitige Gehäuseteil der Unterdrucktherapieeinheit
- 52: vorderseitige Gehäuseteil der Unterdrucktherapieeinheit

### Beschreibung der Figuren

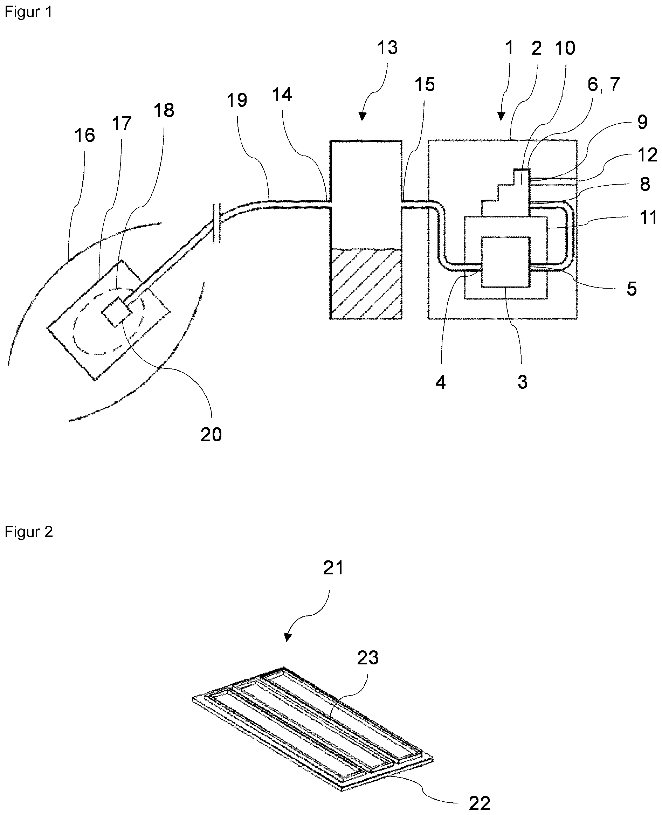

Figur 1 zeigt eine Vorrichtung zur Unterdrucktherapie gemäß des zweiten Aspektes der Erfindung schematisch in einer bevorzugten Ausführungsform. Diese Figur soll hauptsächlich der Veranschaulichung des Fluidpfades der Vorrichtung dienen und zeigt daher nicht alle Merkmale der erfindungsgemäßen Vorrichtung.

Die Vorrichtung umfasst eine Unterdrucktherapieeinheit (1). Die Unterdrucktherapieeinheit (1) umfasst (a) ein Gehäuse (2), (b) eine Pumpe (3) mit einem Eingang (4) und einem Ausgang (5), (c) einen Schalldämpfer (6) mit einem Gehäuse (7), einem Eingang (8), einem Ausgang (9), mehreren Filterkammern (10; nur eine Kammer dargestellt) und einem schalldämpfenden Material (nicht dargestellt), sowie (d) einen Rahmen (11) zur Anordnung und/oder Befestigung von inneren Komponenten der Unterdrucktherapieeinheit (1). Der Schalldämpfer (6) und der Rahmen (11) sind miteinander verbunden. Die erfindungswesentliche Ausgestaltung des Schalldämpfergehäuses wird erst in den nachfolgenden Figuren genauer beschrieben. Wie aus Figur 1 hervorgeht, befinden sich die Pumpe (3), der Schalldämpfer (6) und der Rahmen (11) innerhalb des Gehäuses der Unterdrucktherapieeinheit (2). Der Ausgang der Pumpe (5) steht in fluider Kommunikation mit dem Eingang des Schalldämpfers (8). Der Ausgang des Schalldämpfers (9) steht in fluider Kommunikation mit der die Unterdrucktherapieeinheit (1) umgebenden Atmosphäre. Die fluide Kommunikation kann hergestellt werden, indem ein Schlauch den Pumpenausgang (5) mit dem Schalldämpfereingang (8) verbindet und ein weiterer Schlauch den Schalldämpferausgang (9) mit einem Auslass (12) am Gehäuse der Unterdrucktherapieeinheit verbindet. An das Gehäuse der Unterdrucktherapieeinheit (2) ist ein Behälter zum Sammeln der abgesaugten Wundflüssigkeit (13) mit einem Eingang (14) und einem Ausgang (15) lösbar angebracht. Der Ausgang des Behälters (15) steht in fluider Kommunikation mit dem Eingang der Pumpe (4). Die fluide Kommunikation kann hergestellt werden, indem ein Schlauch an dem Pumpeneingang (4) befestigt und an einem Sauganschluss (nicht dargestellt) im Gehäuse der Unterdrucktherapieeinheit angeschlossen wird. Der Ausgang des Behälters (15) schließt dann direkt an den Sauganschluss an, wenn der Behälter (13) an die Unterdrucktherapieeinheit (1) angekoppelt ist.

Die Vorrichtung aus Figur 1 umfasst weiterhin einen Wundverband (17), welcher die Wunde (18) im Wesentlichen luftdicht gegenüber die den Wundverband umgebende Atmosphäre abschließt, so dass ein Unterdruck innerhalb des Wundverbandes hergestellt und aufrechterhalten werden kann.

Um eine fluide Kommunikation zwischen der Unterdrucktherapieeinheit (1) und dem Wundverband (17) herzustellen, umfasst die Vorrichtung aus Figur 1 ein Verbindungsmittel (19). Bei dem Verbindungsmittel handelt es sich um einen Drainageschlauch, welcher an seinem wundseitigen Ende mit einem Anschlussstück (Port, 20) ausgestattet ist. Der Port (20) wird üblicherweise außen am Wundverband fixiert und steht mit dem Wundraum über eine Öffnung in dem Wundverband in fluider Kommunikation. An seinem geräteseitigen beziehungsweise pumpseitigen Ende ist der Drainageschlauch an dem Eingang des Sammelbehälters (14) angeschlossen.

Wird die Unterdrucktherapieeinheit (1) der Vorrichtung aus Figur 1 in Betrieb genommen, können in der Wunde befindliche Fluide abgesaugt werden. Abgesaugte Wundflüssigkeit kann in dem Sammelbehälter (13) aufgefangen werden. Abgesaugte Gase können über die Pumpe und den der Pumpe nachgeschalteten Schalldämpfer nach außen abgegeben werden.

Die Figur 2 zeigt ein erstes Gehäuseteil (21) des Schalldämpfers in einer bevorzugten Ausführungsform. Das erste Gehäuseteil umfasst eine rechteckige Platte (22). Eine Fläche der Platte (22) umfasst Erhebungen (23) zur Einpassung in das zweite Gehäuseteil.

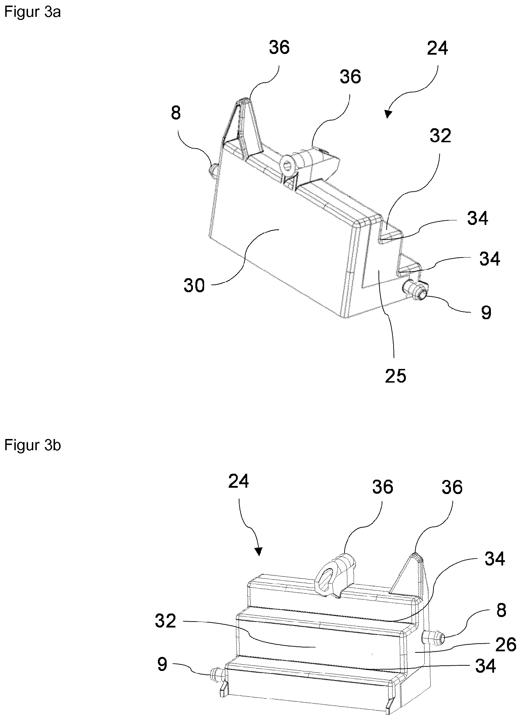

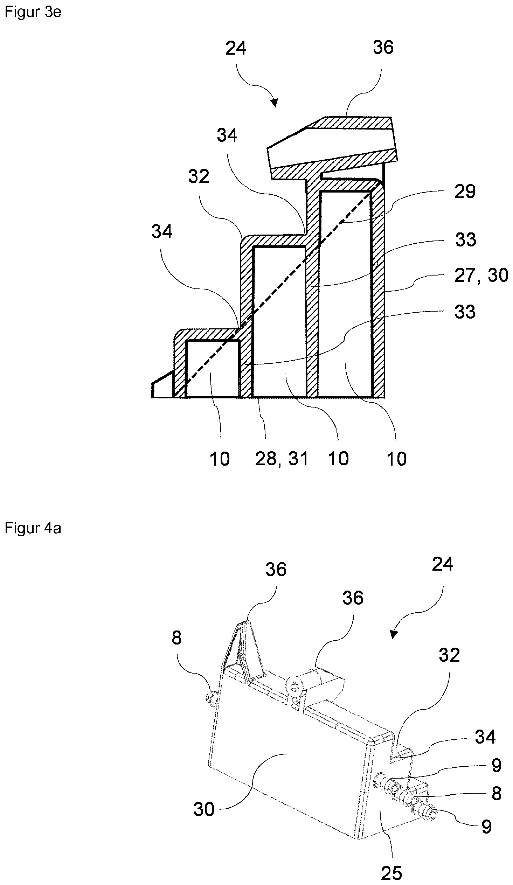

Die Figuren 3a bis 3e zeigen ein zweites Gehäuseteil (24) des Schalldämpfers in einer bevorzugten Ausführungsform. Das zweite Gehäuseteil (24) umfasst einen Hohlkörper in der Form eines geraden Prismas mit einer Grundfläche (25), einer Deckfläche (26) und einer Mantelfläche (30, 31, 32). Die Grundfläche (25) wird auf Basis eines rechtwinkligen Dreiecks mit einer ersten Kathete (27), einer zweiten Kathete (28) und einer imaginären Hypotenuse (29) gebildet. Die Grundfläche (25) weist entlang der imaginären Hypotenuse (29) einen treppenartigen Verlauf mit drei Stufen auf, deren insgesamt sechs Abschnitte entweder parallel oder rechtwinklig zu der ersten oder der zweiten Kathete (27, 28) verlaufen (siehe Querschnittsansicht in Figur 3e). Die Mantelfläche weist dann eine nicht-gestufte erste Seitenfläche (30), eine nicht-gestufte zweite Seitenfläche (31) und eine gestufte dritte Seitenfläche (32) auf. Innerhalb des zweiten Gehäuseteils (24) verlaufen zwei Trennwände (33) parallel zu der ersten Seitenfläche (30) und entlang von zwei Kanten (34) der dritten Seitenfläche (32). Die zweite Seitenfläche (31) ist offen ausgebildet und kann von dem ersten Gehäuseteil (21) aus Figur 2 verschlossen werden, wobei drei quaderförmige Filterkammern unterschiedlicher Größe ausgebildet werden.

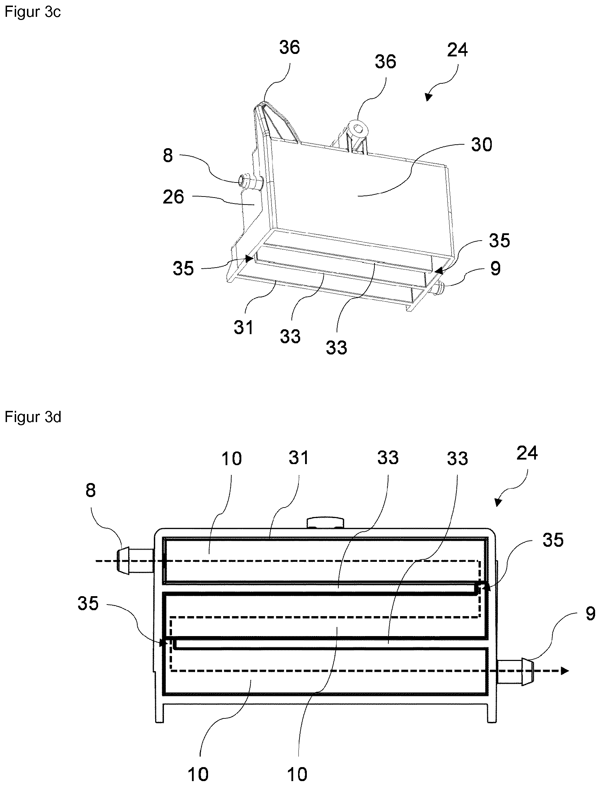

An dem zweiten Gehäuseteil (24) sind auch der Eingang (8) und der Ausgang (9) des Schalldämpfers angeordnet. Der Eingang (8) mündet in die größte der drei Filterkammern und befindet sich an der Deckfläche (26). Der Ausgang (9) mündet in die kleinste der drei Filterkammer und befindet sich an der Grundfläche (25). Sowohl der Eingang (8) als auch der Ausgang (9) sind mit einem Schlauchanschluss ausgestattet. Jede der zwei Trennwände (33) weist eine Öffnung in der Form eines schmalen Spaltes (35) angrenzend an die Grundbeziehungsweise Deckfläche auf. Durch die beschriebene Anordnung von dem Eingang (8), den beiden spaltförmigen Öffnungen in den zwei Trennwänden (35) sowie dem Ausgang (9) liegt ein verschlungener Fluidpfad im Schalldämpfer vor. Der verschlungene Fluidpfad ist in Figur 3d mit einem Pfeil angedeutet. Weiterhin sind an dem zweiten Gehäuseteil (24) auf der Stufe, welche an die erste Seitenfläche (30) angrenzt, zwei ergänzende Gehäusekomponenten (36) vorhanden. Die auf der Stufe mittig angeordnete Komponente (36) dient als Führung für eine Schraube, welche die beiden Gehäuseteile der Unterdrucktherapieeinheit (1) miteinander verbindet.

Die Größe des von dem zweiten Gehäuseteil (24) umfassten Prismas aus den Figuren 3a bis 3e ohne Einbeziehung der zuvor genannten ergänzenden Gehäusekomponenten (36) kann beispielhaft mit den nachfolgenden Angaben genauer beschrieben werden:
Länge der ersten Kathete: 39,5 mm
Länge der zweiten Kathete: 38 mm
Maße der kleinsten Filterkammer (im Querschnitt): 10 mm x 10,5 mm
Maße der mittleren Filterkammer (im Querschnitt): 10 mm x 27,5 mm
Maße der größten Filterkammer (im Querschnitt): 10 mm x 37,5 mm
Höhe des Prismas (= Länge der Seitenkanten): 74 mm
Dicke der Wandungen: 2 mm

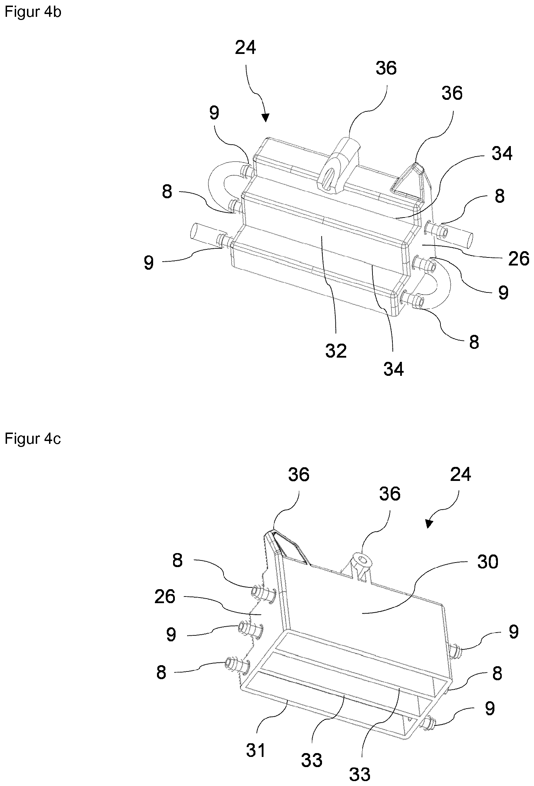

Die Figuren 4a bis 4c zeigen das zweite Gehäuseteil (24) des Schalldämpfers in einer weiteren bevorzugten Ausführungsform. Das zweite Gehäuseteil (24) aus den Figuren 4a bis 4c ist ähnlich wie das zuvor beschriebene zweite Gehäuseteil aufgebaut. Das zweite Gehäuseteil (24) aus den Figuren 4a bis 4c besitzt jedoch keine Öffnungen in den beiden Trennwänden (33, siehe Figur 4c). Stattdessen sind jeder Filterkammer ein Eingang (8) und ein Ausgang (9) zugeordnet (siehe insbesondere Figur 4b). In Figur 4b ist die Verschlauchung der Anschlüsse angedeutet, mit der ein verschlungener Fluidpfad durch die drei Filterkammern erhalten werden kann.

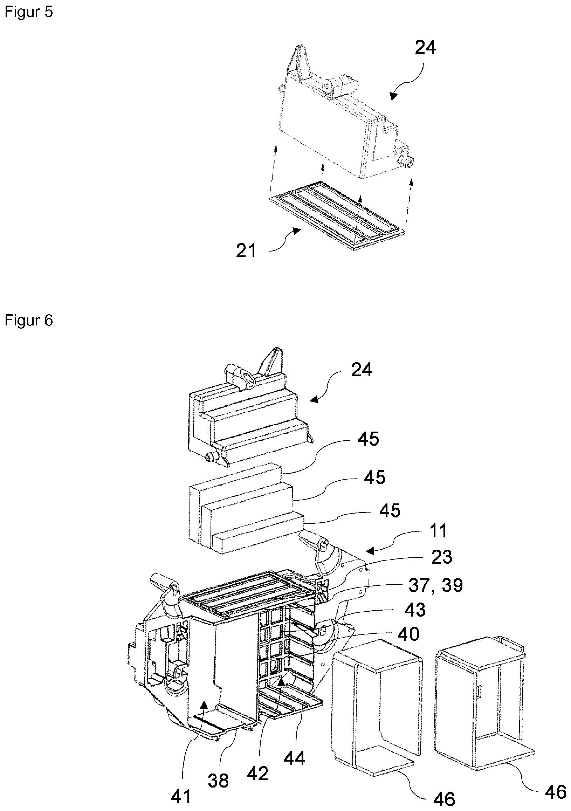

Die Figur 5 zeigt das erste Gehäuseteil (21) nach Figur 2 und das zweite Gehäuseteil (24) nach Figur 3 vor dem Verbinden der Gehäuseteile. Wie aus der Figur hervorgeht kann das zweite Gehäuseteil (24) von dem ersten Gehäuseteil (21) deckelartig verschlossen werden.

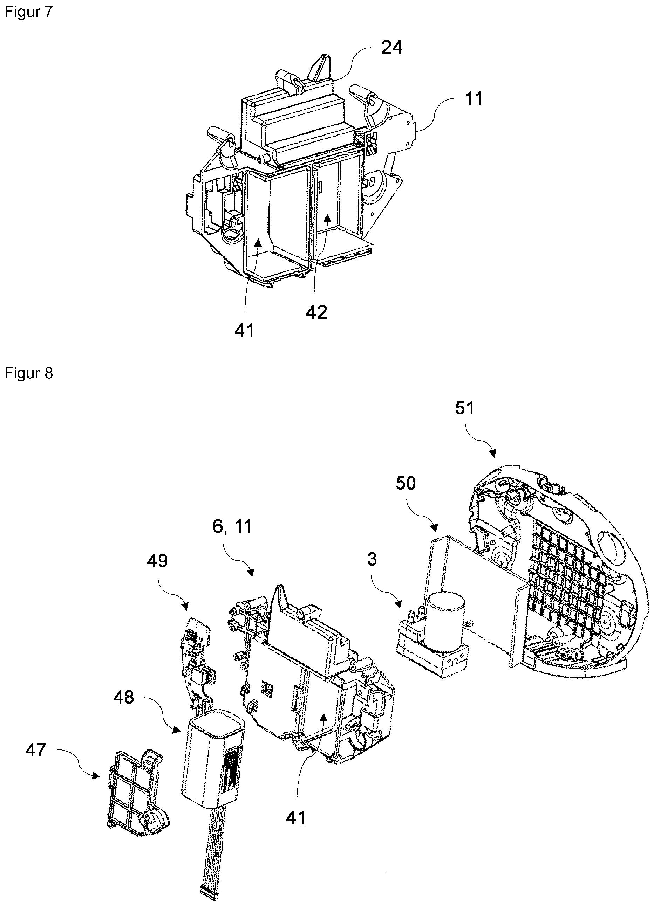

Figuren 6 und 7 zeigen ein in einem Rahmen (11) integriertes erstes Gehäuseteil (37) und das zweite Gehäuseteil (24) nach Figur 3 vor beziehungsweise nach dem Verbinden der Gehäuseteile. Der Rahmen (11) umfasst eine erste Platte (38), eine zweite Platte (39) und eine dritte Platte (40), welche doppel-T-förmig angeordnet sind. Das erste Gehäuseteil (37) des Schalldämpfers ist in die zweite Platte (39) integriert. Die beiden, unter anderem von der ersten, der zweiten und der dritten Platte (38, 39, 40) begrenzten Bereiche (41, 42) werden zur Anordnung beziehungsweise Verstauung eines Akkumulators und der Pumpe (3) verwendet. Der Bereich zur Verstauung der Pumpe (42) weist ausgeschnittene Segmente (43) und schmale Rippen (44) auf. Damit kann die Kontaktfläche des Rahmens (11) mit der Pumpe (3) und infolgedessen die Schallübertragung reduziert werden. In Figur 6 ist auch das schalldämpfende Material (45) dargestellt, mit welchem die Filterkammern vollständig ausgefüllt werden. Bei dem schalldämpfenden Material (45) handelt es sich um drei quaderförmige Blöcke aus beispielsweise einem offenporigen Schaumstoff. Die Bestückung der Filterkammern mit dem schalldämpfenden Material erfolgt, bevor das zweite Gehäuseteil (24) mit dem ersten Gehäuseteil (37) verbunden wird. Das zweite Gehäuseteil (24) wird also zunächst mit dem schalldämpfenden Material (45) befüllt und erst anschließend am Rahmen (11, 37) befestigt. Um die Befestigung des zweiten Gehäuseteils (24) an dem Rahmen (11, 37) zu vereinfachen sowie die Position des zweiten Gehäuseteils (24) an dem Rahmen (11, 37) aufzuzeigen, verfügt das erste Gehäuseteil über Erhebungen (23) zur Umrandung des zweiten Gehäuseteils (24) und zur Einpassung in das zweite Gehäuseteil (24). Zwischen den Erhebungen werden Kanäle auf der Oberfläche des ersten Gehäuseteils (37) ausgebildet. In diese Kanäle können sich die Kanten der offenen Außenfläche (31) des zweiten Gehäuseteils (24) exakt einpassen. In die Kanäle kann auch ein Klebstoff eingebracht werden, welcher für die dauerhafte Verbindung des ersten mit dem zweiten Gehäuseteils eingesetzt wird. Die Kanäle können dann den korrekten Kleberauftrag sicherstellen und ein Wegfließen des noch flüssigen Klebstoffes vor der Verbindung der beiden Gehäuseteile verhindern. In Figur 6 sind weiterhin zwei Schaumstofflagen (46) dargestellt, welche an dem Rahmen zu Schutz-und Dämmzwecken angebracht werden. Derartige Schaumstofflagen werden auch noch an anderen Oberflächen innerhalb der Unterdrucktherapieeinheit angebracht (siehe Komponente 50 in Figur 8).

Das in Figur 6 dargestellte schalldämpfende Material (45) kann beispielhaft mit folgenden weiteren Angaben genauer erläutert werden:
Art des Schaumstoffes: offenporiger, retikulierter Polyurethanschaumstoff
Porendichte: 45 ppi
Luftdurchlässigkeit (gemessen gemäß DIN EN ISO 9237): 3000 +/- 750 l/m²s
Rohdichte (gemessen gemäß DIN EN ISO 845): 28 kg/m³
Abmessungen der drei Schaumstoffquader (Breite x Höhe x Länge): (1) 10 mm x 10 mm x 70 mm; (2) 10 mm x 27 mm x 70 mm; (3) 10 mm x 37 mm x 70 mm

Die Figur 8 zeigt den Rahmen (11) mit dem Schalldämpfer (6) nach Figur 7 und weitere Komponenten der Unterdrucktherapieeinheit (1) in einer bevorzugten Ausführungsform. Bei den in Figur 8 dargestellten Komponenten handelt es sich um: eine Abdeckung des Rahmens (47); den Akkumulator (48); eine Leiterplatte (49); den Rahmen (11) mit Schalldämpfer (6); die Pumpe (3); eine Schaumstofflage (50) zur Befestigung am rückseitigen Gehäuseteil der Unterdrucktherapieeinheit; das rückseitige Gehäuseteil der Unterdrucktherapieeinheit (51).

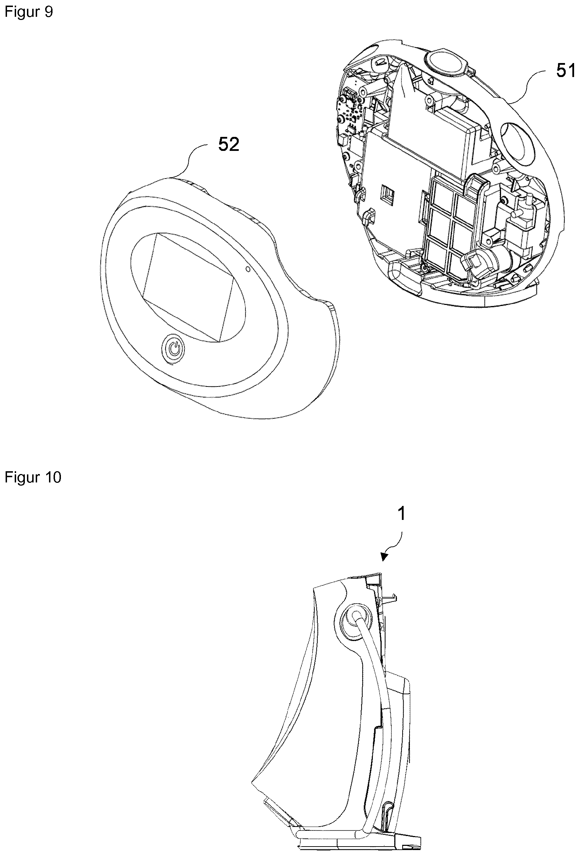

Die Figuren 9 und 10 zeigen die Unterdrucktherapieeinheit (1) in einer bevorzugten Ausführungsform mit den Komponenten aus Figur 8 und mit geöffnetem beziehungsweise verschlossenem Gehäuse (51, 52). Das vorderseitige Gehäuseteil der Unterdrucktherapieeinheit (52) nimmt einen An/Aus-Schalter und einen Berührungsbildschirm sowie weitere, nicht dargestellte Komponenten der Unterdrucktherapieeinheit auf.

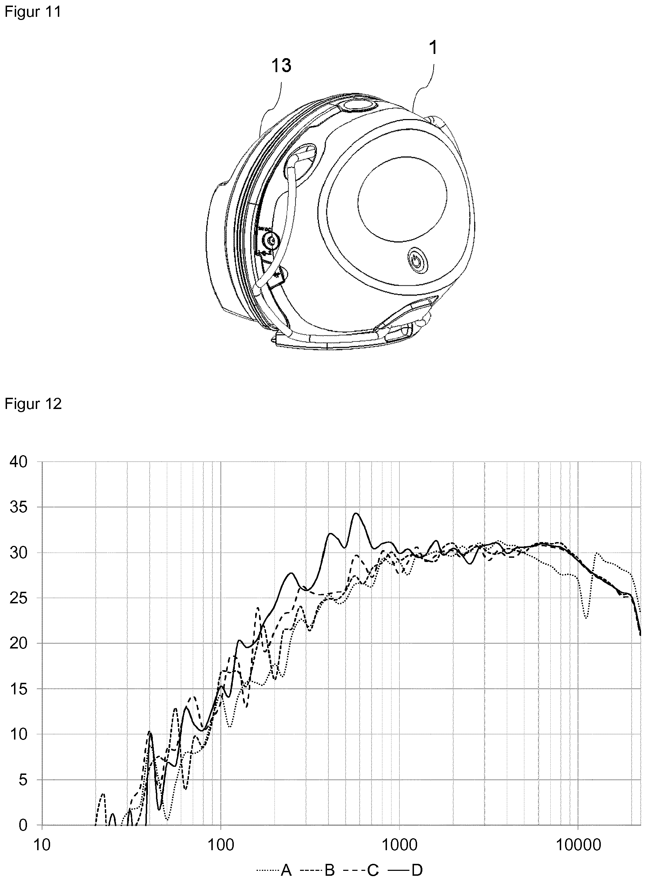

Die Figur 11 zeigt die Unterdrucktherapieeinheit (1) nach Figur 10 mit einem angekoppelten Sammelbehälter (13) für die abgesaugte Wundflüssigkeit in einer bevorzugten Ausführungsform.

### Messungen des Schalldruckpegels (Figur 12)

Eine Unterdrucktherapieeinheit mit einem ähnlichen Aufbau wie in den Figuren 9 und 10 dargestellt wird mit verschiedenen Schalldämpfern betrieben und es werden kalibrierte Schalldruckpegelmessungen in einer reflexionsfreien Kammer durchgeführt. Die Unterdrucktherapieeinheit besitzt als Unterdruckquelle eine elektrische Membranpumpe vom Typ SP622 EC-BL der Firma Schwarzer. Die Schalldruckpegelmessungen werden bei einer Leckagerate von 0,4 l/min durchgeführt und ein Zielwert von 125 mmHg an der Unterdrucktherapieeinheit eingestellt. Der Schalldruckpegel wird 5 cm entfernt von der Vorderseite der Unterdrucktherapieeinheit gemessen. Die Ergebnisse der Schalldruckpegelmessungen sind in Figur 12 dargestellt. Die x-Achse des Diagrammes aus Figur 12 gibt die Frequenz in Hertz (Hz) an. Die y-Achse gibt den Schalldruckpegel in Dezibel (dBA) an. Die vier in dem Diagramm dargestellten Kurven A, B, C und D haben die folgende Bedeutung:
Kurve A: Messdaten des Hintergrundgeräusches in der Messkammer.
Kurve B: Messdaten der Unterdrucktherapieeinheit mit einem zweiten Gehäuseteil des Schalldämpfers wie in Figur 4 dargestellt. Die Größe des zweiten Gehäuseteils entspricht den im Zusammenhang mit Figur 3 gemachten Angaben. Das schalldämpfende Material des Schalldämpfers entspricht den im Zusammenhang mit Figur 6 gemachten Angaben.
Kurve C: Messdaten der Unterdrucktherapieeinheit mit einem zweiten Gehäuseteil des Schalldämpfers wie in Figur 3 dargestellt. Die Größe des zweiten Gehäuseteils entspricht den im Zusammenhang mit Figur 3 gemachten Angaben. Das schalldämpfende Material des Schalldämpfers entspricht den im Zusammenhang mit Figur 6 gemachten Angaben.
Kurve D: Messdaten der Unterdrucktherapieeinheit mit einem aus dem Stand der Technik bekannten Schalldämpfer der Firma Schwarzer Precision (SP). Das Gehäuse des Schalldämpfers weist eine zylindrische Form auf. Die Gesamtlänge des Schalldämpfergehäuses beträgt 80 mm. Der Durchmesser des Schalldämpfergehäuses beträgt 33 mm.

Das menschliche Gehör kann Frequenzen in einem Bereich zwischen 16 Hz und 20 kHz wahrnehmen. Die Schalldruckpegelmessungen zeigen, dass bevorzugte Ausführungsformen des erfindungsgemäßen Schalldämpfers einen verbesserten schalldämpfenden Effekt in diesem Frequenzbereich aufweisen können.

## Patentansprüche

1. Unterdrucktherapieeinheit (1) zur Behandlung von Wunden am menschlichen oder tierischen Körper mittels Unterdruck umfassend
- ein die Unterdrucktherapieeinheit (1) nach außen abgrenzendes Gehäuse (2),
- eine Pumpe (3) zur Erzeugung eines Unterdrucks mit einem Eingang (4) und einem Ausgang (5),
- einen Schalldämpfer (6) für die Pumpe (3) mit einem einen Hohlraum ausbildenden Gehäuse (7), einem schalldämpfenden Material (45), einem Eingang (8) und einem Ausgang (9),
wobei der Ausgang (5) der Pumpe (3) in fluider Kommunikation mit dem Eingang (8) des Schalldämpfers (6) steht und der Ausgang (9) des Schalldämpfers (6) in fluider Kommunikation mit der die Unterdrucktherapieeinheit (1) umgebenden Atmosphäre steht, und
wobei sich die Pumpe (3) und der Schalldämpfer (6) innerhalb des Gehäuses (2) der Unterdrucktherapieeinheit (1) befinden,
**dadurch gekennzeichnet, dass**
das Gehäuse (7) des Schalldämpfers (6) einen Hohlkörper in der Form eines geraden Prismas mit einer Grundfläche (25), einer Deckfläche (26) und einer Mantelfläche (30, 31, 32) umfasst, wobei
- die Grundfläche des Prismas (25) auf Basis eines rechtwinkligen Dreiecks mit einer ersten Kathete (27), einer zweiten Kathete (28) und einer imaginären Hypotenuse (29) gebildet wird, wobei die Grundfläche (25) entlang der imaginären Hypotenuse (29) eine treppenartige Stufung aufweist und wobei die die treppenartige Stufung bildenden Abschnitte der Grundfläche (25) entweder parallel oder rechtwinklig zu der ersten oder der zweiten Kathete (27, 28) verlaufen,
so dass die Mantelfläche des Prismas eine nicht-gestufte erste Seitenfläche (30) und eine nicht-gestufte zweite Seitenfläche (31) und eine gestufte dritte Seitenfläche (32) aufweist, wobei
- eine oder mehrere Trennwände (33), welche parallel zu der ersten Seitenfläche des Prismas (30) und entlang einer Kante (34) der dritten Seitenfläche des Prismas (32) verlaufen, in dem Hohlraum des Prismas angeordnet sind,
so dass der Schalldämpfer (6) im Wesentlichen quaderförmige, aneinander angrenzende Filterkammern (10) unterschiedlicher Größe aufweist.

2. Unterdrucktherapieeinheit (1) nach Anspruch 1, wobei an das Gehäuse (2) der Unterdrucktherapieeinheit (1) ein Behälter (13) zum Sammeln von einer abgesaugten Wundflüssigkeit lösbar anbringbar ist, weicher in fluide Kommunikation mit dem Eingang (4) der Pumpe (3) gebracht werden kann.

3. Unterdrucktherapieeinheit (1) nach Anspruch 1 oder 2, wobei der Hohlkörper ein erstes Gehäuseteil (21) und ein zweites Gehäuseteil (24), welche zur Ausbildung der Filterkammern (10) miteinander verbindbar sind, umfasst.

4. Unterdrucktherapieeinheit (1) nach Anspruch 3, wobei die zweite Seitenfläche des Prismas (31) von dem ersten Gehäuseteil (21) gebildet wird.

5. Unterdrucktherapieeinheit (1) nach Anspruch 4, wobei
- die Grundfläche des Prismas (25) und
- die Deckfläche des Prismas (26) und
- die erste Seitenfläche des Prismas (30) und
- die dritte Seitenfläche des Prismas (32) und
- jede Trennwand (33) und
- vorzugsweise der Eingang (8) und der Ausgang (9) des Schalldämpfers (6) von dem zweiten Gehäuseteil (24) gebildet werden.

6. Unterdrucktherapieeinheit (1) nach einem der Ansprüche 3 bis 5, wobei das erste Gehäuseteil (21) eine oder mehrere Erhebungen (23) zur Umrandung des zweiten Gehäuseteils (24) und/oder zur Einpassung in das zweite Gehäuseteil (24) umfasst.

7. Unterdrucktherapieeinheit (1) nach einem der Ansprüche 3 bis 6, wobei das erste Gehäuseteil (21) und/oder das zweite Gehäuseteil (24) ein Mittel (36), welches zur Befestigung der Gehäuseteile innerhalb der Unterdrucktherapieeinheit vorgesehen ist und/oder zur Befestigung anderer innerer Komponenten der Unterdrucktherapieeinheit vorgesehen ist und/oder eine Führung für ein Befestigungsmittel bildet, umfasst.

8. Unterdrucktherapieeinheit (1) nach einem der vorangehenden Ansprüche, wobei jede Trennwand (33) eine Öffnung (35) aufweist, so dass die Filterkammern (10) in eine fluide Kommunikation miteinander gebracht werden können.

9. Unterdrucktherapieeinheit (1) nach Anspruch 8, wobei
- der Eingang (8) und der Ausgang (9) des Schalldämpfers (6) jeweils an der Grundfläche (25) oder der Deckfläche (26) des Prismas angeordnet sind und jeweils in eine der beiden äußeren Filterkammern (10) münden,
- die Öffnung (35) jeder Trennwand (33) an die Grundfläche (25) oder die Deckfläche (26) des Prismas angrenzt, und
- der Eingang (8) des Schalldämpfers (6), die Öffnung (35) in der Trennwand (33), falls vorhanden die Öffnung (35) jeder weiteren Trennwand (33) und der Ausgang (9) des Schalldämpfers (6) derart gegenüberliegend an der Grundfläche (25) und der Deckfläche (26) des Prismas angeordnet sind beziehungsweise an die Grundfläche (25) und die Deckfläche (26) des Prismas angrenzen, dass ein verschlungener Fluidpfad durch die Filterkammern (10) des Schalldämpfers (6) ausgebildet werden kann.

10. Unterdrucktherapieeinheit (1) nach Anspruch 8 oder 9, wobei die Öffnung (35) in jeder Trennwand (33) dadurch ausgebildet wird, dass die Trennwand (33) nicht durchgehend zwischen der Grundfläche (25) und der Deckfläche (26) des Prismas verläuft, so dass ein Spalt (35) zwischen der Trennwand (33) und der Grundfläche (25) oder der Deckfläche (26) des Prismas verbleibt.

11. Unterdrucktherapieeinheit (1) nach einem der vorangehenden Ansprüche, wobei der Eingang (8) des Schalldämpfers (6) in die größte Filterkammer (10) und der Ausgang (9) des Schalldämpfers (6) in die kleinste Filterkammer (10) münden.

12. Unterdrucktherapieeinheit (1) nach einem der vorangehenden Ansprüche, wobei die Unterdrucktherapieeinheit (1) weiterhin einen Rahmen (11) zur Anordnung und/oder Befestigung von inneren Komponenten der Unterdrucktherapieeinheit umfasst, wobei sich der Rahmen (11) innerhalb des Gehäuses (2) der Unterdrucktherapieeinheit (1) befindet.

13. Unterdrucktherapieeinheit (1) nach Anspruch 12, wobei der Schalldämpfer (6) an dem Rahmen (11) befestigt ist.

14. Unterdrucktherapieeinheit (1) nach einem der Ansprüche 3 bis 11 und einem der Ansprüche 12 oder 13, wobei das erste Gehäuseteil (21) des Schalldämpfers (6) durch einen Abschnitt des Rahmens (11) gebildet wird.

15. Vorrichtung zur Unterdrucktherapie von Wunden am menschlichen oder tierischen Körper, umfassend
- eine Unterdrucktherapieeinheit (1) nach einem der vorangehenden Ansprüche,
- einen Wundverband (17), welcher die Wunde (18) im Wesentlichen luftdicht gegenüber die den Wundverband (17) umgebende Atmosphäre abschließt, so dass ein Unterdruck innerhalb des Wundverbandes (17) hergestellt und aufrechterhalten werden kann, und
- ein Verbindungsmittel (19, 20), welches eine fluide Kommunikation zwischen der Unterdrucktherapieeinheit (1) und dem Wundverband (17) herstellen kann.

## Claims

1. Vacuum therapy unit (1) for the treatment of wounds on the human or animal body by means of vacuum, comprising
- a housing (2) delimiting the vacuum therapy unit (1) to the outside,
- a pump (3) for generating a vacuum, with an inlet (4) and an outlet (5),
- a sound damper (6) for the pump (3), with a housing (7) forming a cavity, and with a sound-damping material (45), an inlet (8) and an outlet (9),
wherein the outlet (5) of the pump (3) is in fluidic communication with the inlet (8) of the sound damper (6), and the outlet (9) of the sound damper (6) is in fluidic communication with the atmosphere surrounding the vacuum therapy unit (1), and wherein the pump (3) and the sound damper (6) are located inside the housing (2) of the vacuum therapy unit (1),
**characterized in that**
the housing (7) of the sound damper (6) comprises a hollow body in the form of a right prism with a base surface (25), a top surface (26) and a lateral surface (30, 31, 32), wherein
- the base surface of the prism (25) is formed on the basis of a right-angled triangle with a first cathetus (27), a second cathetus (28) and an imaginary hypotenuse (29), wherein the base surface (25) has a stairway-like gradation along the imaginary hypotenuse (29), and wherein the portions of the base surface (25) that form the stairway-like gradation extend either parallel or at a right angle to the first or the second cathetus (27, 28),
such that the lateral surface of the prism has a non-stepped first side face (30) and a non-stepped second side face (31) and a stepped third side face (32), wherein
- one or more partition walls (33), which extend parallel to the first side face of the prism (30) and along an edge (34) of the third side face of the prism (32), are arranged in the cavity of the prism,
such that the sound damper (6) has substantially cuboid, mutually adjoining filter chambers (10) of different size.

2. Vacuum therapy unit (1) according to Claim 1, wherein a container (13) for collecting an aspirated wound liquid can be mounted releasably on the housing (2) of the vacuum therapy unit (1) and can be brought into fluidic communication with the inlet (4) of the pump (3).

3. Vacuum therapy unit (1) according to Claim 1 or 2, wherein the hollow body comprises a first housing part (21) and a second housing part (24), which are connectable to each other to form the filter chambers (10).

4. Vacuum therapy unit (1) according to Claim 3, wherein the second side face of the prism (31) is formed by the first housing part (21).

5. Vacuum therapy unit (1) according to Claim 4,
wherein
- the base surface of the prism (25) and
- the top surface of the prism (26) and
- the first side face of the prism (30) and
- the third side face of the prism (32) and
- each partition wall (33) and
- preferably the inlet (8) and the outlet (9) of the sound damper (6)
are formed by the second housing part (24).

6. Vacuum therapy unit (1) according to one of Claims 3 to 5, wherein the first housing part (21) comprises one or more elevations (23) for bordering the second housing part (24) and/or for fitting into the second housing part (24).

7. Vacuum therapy unit (1) according to one of Claims 3 to 6, wherein the first housing part (21) and/or the second housing part (24) comprise (s) a means (36) which is provided to secure the housing parts inside the vacuum therapy unit and/or is provided to secure other internal components of the vacuum therapy unit and/or forms a guide for a securing means.

8. Vacuum therapy unit (1) according to one of the preceding claims, wherein each partition wall (33) has an opening (35), such that the filter chambers (10) can be brought into fluidic communication with one another.

9. Vacuum therapy unit (1) according to Claim 8,
wherein
- the inlet (8) and the outlet (9) of the sound damper (6) are each arranged on the base surface (25) or the top surface (26) of the prism and each open into one of the two outer filter chambers (10),
- the opening (35) of each partition wall (33) adjoins the base surface (25) or the top surface (26) of the prism, and
- the inlet (8) of the sound damper (6), the opening (35) in the partition wall (33), if present the opening (35) of each further partition wall (33), and the outlet (9) of the sound damper (6) are arranged opposite one another on the base surface (25) and the top surface (26) of the prism, or adjoin the base surface (25) and the top surface (26) of the prism, in such a way that a serpentine fluid path can be formed through the filter chambers (10) of the sound damper (6).

10. Vacuum therapy unit (1) according to Claim 8 or 9, wherein the opening (35) in each partition wall (33) is formed by virtue of the fact that the partition wall (33) does not extend continuously between the base surface (25) and the top surface (26) of the prism, such that a gap (35) remains between the partition wall (33) and the base surface (25) or the top surface (26) of the prism.

11. Vacuum therapy unit (1) according to one of the preceding claims, wherein the inlet (8) of the sound damper (6) opens into the largest filter chamber (10), and the outlet (9) of the sound damper (6) opens into the smallest filter chamber (10).

12. Vacuum therapy unit (1) according to one of the preceding claims, wherein the vacuum therapy unit (1) moreover comprises a frame (11) for arranging and/or securing internal components of the vacuum therapy unit, wherein the frame (11) is located inside the housing (2) of the vacuum therapy unit (1) .

13. Vacuum therapy unit (1) according to Claim 12, wherein the sound damper (6) is secured to the frame (11) .

14. Vacuum therapy unit (1) according to one of Claims 3 to 11 and either of Claims 12 and 13, wherein the first housing part (21) of the sound damper (6) is formed by a portion of the frame (11).

15. Device for vacuum therapy of wounds on the human or animal body, comprising
- a vacuum therapy unit (1) according to one of the preceding claims,
- a wound dressing (17), which closes the wound (18) off in a substantially airtight manner from the atmosphere surrounding the wound dressing (17), such that a vacuum can be produced and maintained inside the wound dressing (17), and
- a connection means (19, 20), which can produce a fluidic communication between the vacuum therapy unit (1) and the wound dressing (17).

## Revendications

1. Unité de thérapie par pression négative (1) pour le traitement de plaies du corps humain ou animal au moyen d'une dépression, comprenant :
- un boîtier (2) délimitant vers l'extérieur l'unité de thérapie par pression négative (1),
- une pompe (3) pour générer une dépression, comprenant une entrée (4) et sortie (5),
- un amortisseur de bruit (6) pour la pompe (3), avec un boîtier (7) constituant une cavité, un matériau amortisseur de bruit (45), une entrée (8) et une sortie (9),
la sortie (5) de la pompe (3) étant en communication fluidique avec l'entrée (8) de l'amortisseur de bruit (6) et la sortie (9) de l'amortisseur de bruit (6) étant en communication fluidique avec l'atmosphère entourant l'unité de thérapie par pression négative (1), et
la pompe (3) et l'amortisseur de bruit (6) se trouvant à l'intérieur du boîtier (2) de l'unité de thérapie par pression négative (1),
**caractérisée en ce que**
le boîtier (7) de l'amortisseur de bruit (6) comprend un corps creux en forme de prisme droit avec une surface de base (25), une surface de dessus (26) et une surface d'enveloppe (30, 31, 32), où
- la surface de base du prisme (25) est formée sur la base d'un triangle rectangle avec un premier côté (27), un deuxième côté (28) et une hypoténuse imaginaire (29), la surface de base (25) présentant, le long de l'hypoténuse imaginaire (29), un gradin en forme d'escalier et les portions de la surface de base (25) formant le gradin en forme d'escalier s'étendant soit parallèlement soit à angle droit par rapport au premier ou au deuxième côté (27, 28),
de telle sorte que la surface d'enveloppe du prisme présente une première surface latérale non étagée (30), une deuxième surface latérale non étagée (31) et une troisième surface latérale étagée (32), où
- une ou plusieurs parois de séparation (33) qui s'étendent parallèlement à la première surface latérale du prisme (30) le long d'une arête (34) de la troisième surface latérale du prisme (32) sont disposées dans la cavité du prisme,
de telle sorte que l'amortisseur de bruit (6) présente des chambres de filtre (10) adjacentes les unes aux autres, essentiellement parallélépipédiques, de tailles différentes.

2. Unité de thérapie par pression négative (1) selon la revendication 1, dans laquelle un récipient (13) pour recueillir un liquide de plaie aspiré peut être monté de manière amovible sur le boîtier (2) de l'unité de thérapie par pression négative (1), lequel peut être amené en communication fluidique avec l'entrée (4) de la pompe (3).

3. Unité de thérapie par pression négative (1) selon la revendication 1 ou 2, dans laquelle le corps creux comprend une première partie de boîtier (21) et une deuxième partie de boîtier (24) qui peuvent être raccordées l'une à l'autre pour réaliser les chambres de filtre (10).

4. Unité de thérapie par pression négative (1) selon la revendication 3, dans laquelle la deuxième surface latérale du prisme (31) est formée par la première partie de boîtier (21).

5. Unité de thérapie par pression négative (1) selon la revendication 4, dans laquelle
- la surface de base du prisme (25) et
- la surface de dessus du prisme (26) et
- la première surface latérale du prisme (30) et
- la troisième surface latérale du prisme (32) et
- chaque paroi de séparation (33) et
- de préférence l'entrée (8) et la sortie (9) de l'amortisseur de bruit (6) sont formées par la deuxième partie de boîtier (24).

6. Unité de thérapie par pression négative (1) selon l'une quelconque des revendications 3 à 5, dans laquelle la première partie de boîtier (21) comprend un ou plusieurs rehaussements (23) destinés à border la deuxième partie de boîtier (24) et/ou à être insérés dans la deuxième partie de boîtier (24).

7. Unité de thérapie par pression négative (1) selon l'une quelconque des revendications 3 à 6, dans laquelle la première partie de boîtier (21) et/ou la deuxième partie de boîtier (24) comprennent un moyen (36) qui est prévu pour fixer les parties de boîtier à l'intérieur de l'unité de thérapie par pression négative et/ou qui est prévu pour fixer d'autres composants intérieurs de l'unité de thérapie par pression négative et/ou qui forme un guide pour un moyen de fixation.

8. Unité de thérapie par pression négative (1) selon l'une quelconque des revendications précédentes, dans laquelle chaque paroi de séparation (33) présente une ouverture (35), de telle sorte que les chambres de filtre (10) puissent être amenées en communication fluidique les unes avec les autres.

9. Unité de thérapie par pression négative (1) selon la revendication 8, dans laquelle
- l'entrée (8) et la sortie (9) de l'amortisseur de bruit (6) sont disposées à chaque fois au niveau de la surface de base (25) ou de la surface de dessus (26) du prisme et débouchent à chaque fois dans l'une des deux chambres de filtre (10) extérieures,
- l'ouverture (35) de chaque paroi de séparation (33) est adjacente à la surface de base (25) ou à la surface de dessus (26) du prisme et
- l'entrée (8) de l'amortisseur de bruit (6), l'ouverture (35) dans la paroi de séparation (33), au cas où elle est présente l'ouverture (35) de chaque paroi de séparation supplémentaire (33), et la sortie (9) de l'amortisseur de bruit (6) sont disposées au niveau de la surface de base (25) et de la surface de dessus (26) du prisme en regard les unes des autres, ou sont respectivement adjacentes à la surface de base (25) et à la surface de dessus (26) du prisme, de telle sorte qu'un chemin fluidique tortueux puisse être réalisé par les chambres de filtre (10) de l'amortisseur de bruit (6).

10. Unité de thérapie par pression négative (1) selon la revendication 8 ou 9, dans laquelle l'ouverture (35) dans chaque paroi de séparation (33) est réalisée par le fait que la paroi de séparation (33) ne s'étend pas en continu entre la surface de base (25) et la surface de dessus (26) du prisme, de telle sorte qu'une fente (35) subsiste entre la paroi de séparation (33) et la surface de base (25) ou la surface de dessus (26) du prisme.

11. Unité de thérapie par pression négative (1) selon l'une quelconque des revendications précédentes, dans laquelle l'entrée (8) de l'amortisseur de bruit (6) débouche dans la plus grande chambre de filtre (10) et la sortie (9) de l'amortisseur de bruit (6) débouche dans la plus petite chambre de filtre (10).

12. Unité de thérapie par pression négative (1) selon l'une quelconque des revendications précédentes, dans laquelle l'unité de thérapie par pression négative (1) comprend en outre un cadre (11) pour l'agencement et/ou la fixation de composants internes de l'unité de thérapie par pression négative, le cadre (11) se trouvant à l'intérieur du boîtier (2) de l'unité de thérapie par pression négative (1).

13. Unité de thérapie par pression négative (1) selon la revendication 12, dans laquelle l'amortisseur de bruit (6) est fixé au cadre (11).

14. Unité de thérapie par pression négative (1) selon l'une quelconque des revendications 3 à 11, et selon l'une quelconque des revendications 12 ou 13, dans laquelle la première partie de boîtier (21) de l'amortisseur de bruit (6) est formée par une portion du cadre (11).

15. Dispositif pour une thérapie par pression négative de plaies du corps humain ou animal, comprenant
- une unité de thérapie par pression négative (1) selon l'une quelconque des revendications précédentes,
- un pansement (17) qui ferme la plaie (18) de manière essentiellement étanche à l'air par rapport à l'atmosphère entourant le pansement (17), de telle sorte qu'une pression négative à l'intérieur du pansement (17) puisse être établie et maintenue, et
- un moyen de liaison (19, 20) qui peut établir une communication fluidique entre l'unité de thérapie par pression négative (1) et le pansement (17).
